# EUROPEAN PATENT APPLICATION

(11) **EP 4 372 101 A1**
(43) Date of publication of application: **22.05.2024**
(21) Application number: 23207286.8
(22) Date of filing: 01.11.2023
(51) Int. Cl.: C12Q 1/6869, C12Q 1/6806

(54) **METHODS AND DEVICES OF GENERATING CLUSTERS OF AMPLICONS**

(30) Priority: 02.11.2022 US 202263421693 P
(71) Applicant: EsBiolab LLC, San Diego, CA 92121 (US)
(72) Inventor: QIANG, Liangliang, California, 92121 (US); LI, Wei, California, 92121 (US); HAN, Aijie, California, 92121 (US)
(74) Representative: Leonard, Thomas Charles

(57) **Abstract**

The present disclosure describes methods and microfluidic devices for generating clusters of amplicons for a nucleic acid library, and their uses for high-throughput DNA sequencing or detection of target polynucleotides in a sample.

## Description

### BACKGROUND

Surface DNA oligo or DNA library amplification methods, such as bridge amplification (bPCR), rolling cycle amplification, and Recombinase Polymerase Amplification (RPA), are widely used in DNA sequencing platforms. It is a key step to amplify the signal for DNA sequencing and to improve the signal-to-noise ratio. However, most methods are unable to increase the density and the signal intensity of the amplified DNA clusters at the same time, which limits the density of clusters on the surface and the throughput for high-throughput DNA sequencing applications.

There remains a need to develop new methods for generating clusters of amplicons with both high density and signal intensity for applications such as high-throughput DNA sequencing.

### SUMMARY

The present disclosure is based in part on the development of new methods of generating clusters of amplicons for a nucleic acid library using uniquely designed single-strand primers immobilized on a solid surface. The generated clusters of amplicons can be used for high-throughput DNA sequencing, DNA hybridization to detect target polynucleotides, etc.

In some aspects, provided herein is a method of generating clusters of amplicons for a nucleic acid library, comprising: (a) providing a solid surface immobilized with a plurality of forward primers and a plurality of reverse primers, wherein (i) each forward primer comprises, in 5' to 3' order, a 5' region and a 3' region; (ii) each reverse primer comprises, in 5' to 3' order, a 5' region and a 3' region, and (iii) the 5' region of the forward primer and the 5' region of the reverse primer are fully or partially complementary to each other; (b) contacting the nucleic acid library with the solid surface, wherein each nucleic acid molecule of the library comprises, in 5' to 3' order: (i) a first adapter comprising a sequence that corresponds to the sequence of the 3' region of the reverse primer, a template sequence, and a second adapter comprising a sequence that is complementary to the 3' region of the forward primer, or (ii) a first adapter comprising a sequence that corresponds to the sequence of the 3' region of the forward primer, a template sequence, and a second adapter comprising a sequence that is complementary to the 3' region of the reverse primer; and (c) performing an amplification process on the solid surface to generate the clusters of amplicons, whereby each nucleic acid molecule from the nucleic acid library is amplified into a clonal cluster of amplicons.

Numerous embodiments are further provided that can be applied to any aspect of the present invention and/or combined with any other embodiment described herein. For example, in some embodiments, the method further comprises immobilizing the plurality of forward primers and the plurality of reverse primers to the solid surface prior to step (a). In some embodiments, the solid surface is a flow cell, a solid bead, a glass slide, or a multi-well plate.

In some embodiments, the method further comprises preparing the nucleic acid library prior to step (b). In some embodiments, preparing the nucleic acid library comprises: (i) obtaining nucleic acids from a biological sample, (ii) fragmenting the nucleic acids to proper sizes, and (iii) adding adapters to both ends of the nucleic acid fragments such that the adapter at one end comprises a sequence that corresponds to or is complementary to the 3' region of the forward primer, and the adapter at the other end comprises a sequence that corresponds to or is complementary to the 3' region of the reverse primer.

In some embodiments, the nucleic acid library comprises at least two nucleic acid molecules that have neither the same nor the complementary sequence. In some embodiments, the nucleic acid library is a DNA library. In some embodiments, the DNA library comprises single-strand DNA molecules. In some embodiments, the DNA library comprises double-strand DNA molecules. In some embodiments, the DNA library is a cDNA library or a genomic DNA library.

In some embodiments, the method further comprises denaturing the nucleic acid library prior to step (c).

In some embodiments, the amplification process is a polymerase chain reaction (PCR) or a recombinase polymerase amplification (RPA).

In some embodiments, step (c) comprises: (i) annealing the nucleic acid molecules from the library to the immobilized forward or reverse primers, (ii) generating immobilized DNA strands that are complementary to the nucleic acid molecules from the library by extending the forward or reverse primers, (iii) denaturing the double-strand nucleic acids, and (iv) washing away the nucleic acid molecules that are not immobilized to the solid surface.

In some embodiments, step (c) further comprises one or more cycles of bridge PCR during which the immobilized DNA strand extended from the forward primer hybridizes with the reverse primer to generate the complementary strand, and/or the immobilized DNA strand extended from the reverse primer hybridizes with the forward primer to generate the complementary strand.

In some embodiments, the one or more cycles of bridge PCR generate: (1) an immobilized DNA strand extended from the immobilized forward primer which comprises, in 5' to 3' order, the 5' region of the forward primer, a sequence that corresponds to or is to a nucleic acid molecule of the nucleic acid library, and a sequence that is complementary to the 5' region of the reverse primer or to the 3' portion of the 5' region of the reverse primer; and (2) an immobilized DNA strand extended from the immobilized reverse primer which comprises, in 5' to 3' order, the 5' region of the reverse primer, a sequence that corresponds to or is complementary to a nucleic acid molecule of the DNA library, and a sequence that is complementary to the 5' region of the forward primer or to the 3' portion of the 5' region of the forward primer.

In some embodiments, step (c) further comprises a supersurface PCR during which 3' end of the immobilized DNA strand extended from the forward primer hybridizes with 3' end of the immobilized DNA strand extended from the reverse primer, and each of the two immobilized DNA strands extends at its 3' end using the other immobilized DNA strand as a template.

In some embodiments, step (c) comprises 5 to 75 cycles of PCR, e.g., 15 to 50 cycles of PCR. In some embodiments, the average cluster signal intensity is about 35-50 after 15 cycles of PCR.

In some embodiments, the method further comprises, after step (c): (d) incubating the solid surface with an exonuclease for a period of time; and (e) conducting a second phase of polymerase chain reaction (PCR) to generate the clusters of amplicons. In some embodiments, the exonuclease is selected from exonuclease I, exonuclease T, exonuclease P1, and exonuclease VII. In some embodiments, unused forward primers and reverse primers are removed from the solid surface by the exonuclease at step (d). In some embodiments, step (e) comprises a supersurface PCR during which 3' end of the immobilized DNA strand extended from the forward primer hybridizes with 3' end of the immobilized DNA strand extended from the reverse primer, and each of the two immobilized DNA strands extends at the 3' end using the other immobilized DNA strand as a template. In some embodiments, step (e) comprises 5-50 cycles of PCR, e.g., 25 cycles of PCR.

In some embodiments, step (c) and step (e) comprise a total of 30-60 cycles of PCR. In some embodiments, step (c) comprises 15 cycles of PCR and step (e) comprises 25 cycles of PCR. In some embodiments, the average cluster signal intensity is about 75-100 after 15 cycles of PCR at step (c) and 25 cycles of PCR at step (e).

In some embodiments, the method further comprises sequencing the clusters of amplicons to obtain sequence information for the nucleic acid library. In some embodiments, the method further comprises hybridizing the clusters of amplicons with a probe to detect a target polynucleotide from the nucleic acid library.

In some aspects, provided herein is a method of sequencing a nucleic acid sample comprising: (a) preparing a nucleic acid library from the nucleic acid sample; (b) generating clusters of amplicons using methods described herein; and (c) sequencing the clusters of amplicons to obtain sequence information for the nucleic acid sample.

In some aspects, provided herein is a method of detecting a target polynucleotide from a nucleic acid sample comprising: (a) preparing a nucleic acid library from the nucleic acid sample; (b) generating clusters of amplicons using methods described herein; and (c) hybridizing the clusters of amplicons with a probe to detect a target polynucleotide from the nucleic acid sample.

In some embodiments, the nucleic acid sample is a total RNA sample or an mRNA sample, and step (a) comprises preparing a cDNA library from the total RNA or the mRNA sample.

In some aspects, provided herein is a microfluidic device comprising a solid surface immobilized with a plurality of forward primers and a plurality of reverse primers, wherein (i) each forward primer comprises, in 5' to 3' order, a 5' region and a 3' region; (ii) each reverse primer comprises, in 5' to 3' order, a 5' region and a 3' region, and (iii) the 5' region of the forward primer and the 5' region of the reverse primer are fully or partially complementary to each other. In some embodiments, the solid surface is a flow cell.

As described above, numerous embodiments are further provided that can be applied to any aspect of the present invention and/or combined with any other embodiment described herein. For example, in some embodiments, the forward and/or reverse primers are immobilized to the solid surface at a density of about 1 fmol/mm² to about 1000 fmol/mm², for examples, at a density of about 200 fmol/mm². In some embodiments, the plurality of forward primers and the plurality of reverse primers are immobilized to the solid surface adjacent to each other. In some embodiments, the number of forward primers and the number of reverse primers are at a ratio of from about 1:20 to about 20:1, from about 1:9 to about 9:1, or from about 1:2 to about 2: 1. In certain embodiments, the number of forward primers and the number of reverse primers are at a ratio of about 1:1.

In some embodiments, the 5' region of the forward primer is 3-100 nucleotides in length. In some embodiments, the 5' region of the reverse primer is 3-100 nucleotides in length. In some embodiments, the 5' region of the forward primer and the 5' region of the reverse primer have the same length. In some embodiments, the 5' region of the forward primer and the 5' region of the reverse primer have different lengths.

In some embodiments, the 5' region of the forward primer and the 5' region of the reverse primer are completely complementary to each other. In some embodiments, the 5' region of the forward primer and the 5' region of the reverse primer are partially complementary to each other with at least one mismatch. In some embodiments, the 5' region of the forward primer and the 5' region of the reverse primer are partially complementary to each other with one, two, or three mismatches.

In some embodiments, the 5' region of the forward primer and/or the 5' region of the reverse primer comprise one or more stretches of identical bases. In some embodiments, each of the one or more stretches comprises at least three identical bases. In some embodiments, the 5' region of the forward primer is a poly-A strand and the 5' region of the reverse primer is a poly-T strand, or the 5' region of the forward primer is a poly-T strand and the 5' region of the reverse primer is a poly-A strand.

In some embodiments, the 3' region of the forward primer is 20-50 nucleotides in length. In some embodiments, the 3' region of the forward primer is 24 nucleotides in length. In some embodiments, the 3' region of the forward primer has a nucleotide sequence of 5'-CAAGCAGAAGACGGCATACGAGAT-3' (SEQ ID NO: 1). In some embodiments, the 3' region of the reverse primer is 20-50 nucleotides in length. In some embodiments, the 3' region of the reverse primer is 29 nucleotides in length. In some embodiments, the 3' region of the reverse primer has a nucleotide sequence of 5'-AATGAUACGGCGACCACCGAGATCTACAC-3' (SEQ ID NO: 2). In some embodiments, the 5' region of the forward primer is 20 nucleotides in length and the 5' region of the reverse primer is 10 nucleotides in length. For example, the 5' region of the forward primer consists of 20 A and the 5' region of the reverse primer consists of 10 T, or the 5' region of the forward primer consists of 20 T and the 5' region of the reverse primer consists of 10 A. In some embodiments, the forward primer has a nucleotide sequence of 5'-AAAAAAAAAAAAAAAAAAAACAAGCAGAAGACGGCATACGAGAT-3' (SEQ ID NO: 3); and the reverse primer has a nucleotide sequence of 5'-TTTTTTTTTTAATGAUACGGCGACCACCGAGATCTACAC-3' (SEQ ID NO: 4).

In some embodiments, the plurality of forward primers all have the same nucleotide sequence. In some embodiments, the plurality of reverse primers all have the same nucleotide sequence.

In some aspects, provided herein is a method of producing a microfluidic device described herein.

In some aspects, provided herein is a method of using a microfluidic device described herein to generate clusters of amplicons for a nucleic acid library.

In some aspects, provided herein is a method of using a microfluidic device described herein to sequence a nucleic acid sample.

In some aspects, provided herein is a method of using a microfluidic device described herein to detect a target polynucleotide from a nucleic acid sample.

### BRIEF DESCRIPTION OF FIGURES

**FIG. 1** shows surface DNA oligo amplification by bridge PCR (bPCR).
**FIG. 2** shows E1 and E2 primers with polyA and polyT tail, respectively. Figure discloses SEQ ID NOS 6-7, respectively, in order of appearance.
**FIG. 3** shows the seeding step of library, the library extension, and the 1^{st} cycle of supersurface PCR (ssPCR).
**FIG. 4** shows supersurface PCR after the initial extension and the 1^{st} cycle of PCR.
**FIG. 5** shows supersurface PCR after exonuclease I treatment.
**FIG. 6A** shows average cluster signal intensities from green and red fluorescent channels after 15 cycles of ssPCR and bPCR.
**FIG. 6B** shows max cluster intensities cloud plots for normal bridge PCR amplified clusters and supersurface PCR generated clusters.
**FIG. 7** shows average cluster signal intensities from green and red fluorescent channels after 15 + 25 cycles of ssPCR and bPCR.

### DETAILED DESCRIPTION

### General

Provided herein are methods of generating clusters of amplicons for a nucleic acid library using uniquely designed primers immobilized on a solid surface, microfluidic devices comprising such uniquely designed primers immobilized on a solid surface, and uses of such methods or microfluidic devices to amplify, sequence, detect, and/or quantify one or more nucleic acids in a sample. Such methods enable generation of clusters of amplicons with both higher copies of the targeted nucleic acids and smaller footprints on the solid surface. Using the methods described herein, better signal-to-noise ratio and higher density of clusters of amplicons can be achieved at same time.

### Definitions

The articles "*a*" and "*an*" are used herein to refer to one or to more than one (*e.g.*, to at least one) of the grammatical object of the article. By way of example, "an element" means one element or more than one element.

The term "*binding*" or "*interacting*" refers to an association, which may be a stable association, between two molecules, *e.g*., between a probe and a target nucleic acid molecule, or between a primer and a nucleic acid template, *e.g*., due to, for example, electrostatic, hydrophobic, ionic and/or hydrogen-bond interactions.

As used herein, two nucleic acid sequences "*complement*" one another or are "*complementary*" to one another if they base pair one another at each position.

As used herein, two or more nucleic acid sequences "*correspond*" to each other if they are all complementary to the same nucleic acid sequence. In some embodiments, the two or more nucleic acid sequences that correspond to each other all have the same nucleic acid sequences. In some embodiments, the two or more nucleic acid sequences that correspond to each other may have different nucleic acid sequences. For example, 5'-TTT-3' and 5'-LTCTCT-3' have different nucleic acid sequences but correspond to each other because they are both complementary to 5'-AAA-3'.

As used herein, the *Tm* or *melting temperature* of two oligonucleotides is the temperature at which 50% of the oligonucleotide/targets are bound and 50% of the oligonucleotide/targets are not bound. Tm values of two oligonucleotides are oligonucleotide concentration dependent and are affected by the concentration of monovalent, divalent cations in a reaction mixture. Tm can be determined empirically or calculated using the nearest neighbor formula, as described in Santa Lucia, J. PNAS (USA) 95:1460-1465 (1998), which is hereby incorporated by reference.

As used herein, the term "*primer*" refers to a short (*e.g.*, less than 150 bases) single stranded nucleic acid molecule designated specifically for (e.g., binding specifically to) a particular target polynucleotide (e.g., a nucleic acid molecule from the nucleic acid library described herein). In some embodiments, the primer may comprise DNA, RNA, and/or modified nucleotides such as LNA, PNA, PTO, ZNA, INA, UNA, methylation, 2-O-methyl, halogenated, superbases, iso-dN, inverted bases, L-ribose, etc. In some embodiments, the primer described herein may comprise, at its 5' end, 3' end, and/or internally, one or more chemical modifications that are used to immobilize the primer to a solid surface.

The terms "*polynucleotide*" and "*nucleic acid"* are used herein interchangeably. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, synthetic polynucleotides, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified, such as by conjugation with a labeling component.

### Methods

In some aspects, provided herein is a method of generating clusters of amplicons for a nucleic acid library, comprising: (a) providing a solid surface immobilized with a plurality of forward primers and a plurality of reverse primers, wherein (i) each forward primer comprises, in 5' to 3' order, a 5' region and a 3' region; (ii) each reverse primer comprises, in 5' to 3' order, a 5' region and a 3' region, and (iii) the 5' region of the forward primer and the 5' region of the reverse primer are fully or partially complementary to each other; (b) contacting the nucleic acid library with the solid surface, wherein each nucleic acid molecule of the library comprises, in 5' to 3' order: (i) a first adapter comprising a sequence that corresponds to the sequence of the 3' region of the reverse primer, a template sequence, and a second adapter comprising a sequence that is complementary to the 3' region of the forward primer, or (ii) a first adapter comprising a sequence that corresponds to the sequence of the 3' region of the forward primer, a template sequence, and a second adapter comprising a sequence that is complementary to the 3' region of the reverse primer; and (c) performing an amplification process on the solid surface to generate the clusters of amplicons, whereby each nucleic acid molecule from the nucleic acid library is amplified into a clonal cluster of amplicons.

In some aspects, provided herein is a method of sequencing a nucleic acid sample comprising: (a) preparing a nucleic acid library from the nucleic acid sample; (b) generating clusters of amplicons using methods described herein; and (c) sequencing the clusters of amplicons to obtain sequence information for the nucleic acid sample.

In some aspects, provided herein is a method of detecting a target polynucleotide from a nucleic acid sample comprising: (a) preparing a nucleic acid library from the nucleic acid sample; (b) generating clusters of amplicons using methods described herein; and (c) hybridizing the clusters of amplicons with a probe to detect a target polynucleotide from the nucleic acid sample.

In some embodiments, the nucleic acid sample is a total RNA sample or an mRNA sample, and step (a) comprises preparing a cDNA library from the total RNA or the mRNA sample.

### Immobilized Forward and Reverse Primers

In some embodiments, the method further comprises immobilizing the plurality of forward primers and/or the plurality of reverse primers to the solid surface prior to step (a).

In some embodiments, the forward and/or the reverse primers are uniformly immobilized to the solid surface. In some embodiments, when primers are uniformly attached to the solid surface, the amplification process is stopped before clusters merge into each other in order for detection. In some embodiments, the forward and/or reverse primers are immobilized to the solid surface as an array of primer clusters. In some embodiments, the primer clusters are spatially separated from each other on the solid surface. Preferably, primers are conjugated densely enough to allow the intended amplicon to grow from one primer, anneal to the other primer, and allowing the polymerization of its complementary stand. For example, the forward primers and the reverse primers may be immobilized to the solid surface adjacent to each other to allow the intended amplicon to grow from the forward primer and anneal to the reverse primer, or to grow from the reverse primer and anneal to the forward primer, and allow the polymerization of its complementary stand.

In some embodiments, the forward and/or the reverse primers are immobilized on a 2-dimensional surface. In some such embodiments, the forward and/or the reverse primers are immobilized to the solid surface at a density of about 1 fmol/mm² to about 1000 fmol/mm², for examples, from 10 fmol/mm² to about 1000 fmol/mm², 50 fmol/mm² to about 1000 fmol/mm², 100 fmol/mm² to about 1000 fmol/mm², 500 fmol/mm² to about 1000 fmol/mm², from 1 fmol/mm² to about 500 fmol/mm², 1 fmol/mm² to about 100 fmol/mm², 1 fmol/mm² to about 50 fmol/mm², or 1 fmol/mm² to about 10 fmol/mm². In some embodiments, the forward and/or the reverse primers are immobilized to the solid surface at a density of about 1 fmol/mm², 10 fmol/mm², 20 fmol/mm², 30 fmol/mm², 40 fmol/mm², 50 fmol/mm², 60 fmol/mm², 70 fmol/mm², 80 fmol/mm², 90 fmol/mm², 100 fmol/mm², 200 fmol/mm², 300 fmol/mm², 400 fmol/mm², 500 fmol/mm², 600 fmol/mm², 700 fmol/mm², 800 fmol/mm², 900 fmol/mm², or 1000 fmol/mm². In certain embodiments, the forward and/or the reverse primers are immobilized to the solid surface at a density of about 200 fmol/mm².

In some embodiments, the forward and/or the reverse primers are immobilized on a 3-dimensional surface. In some such embodiments, the forward and/or the reverse primers are immobilized to the solid surface at a density of about 1 fmol/mm³ to about 1000 fmol/mm³, for examples, from 10 fmol/mm³ to about 1000 fmol/mm³, 50 fmol/mm³ to about 1000 fmol/mm³, 100 fmol/mm³ to about 1000 fmol/mm³, 500 fmol/mm³ to about 1000 fmol/mm³, from 1 fmol/mm³ to about 500 fmol/mm³, 1 fmol/mm³ to about 100 fmol/mm³, 1 fmol/mm³ to about 50 fmol/mm³, or 1 fmol/mm³ to about 10 fmol/mm³. In some embodiments, the forward and/or the reverse primers are immobilized to the solid surface at a density of about 1 fmol/mm³, 10 fmol/mm³, 20 fmol/mm³, 30 fmol/mm³, 40 fmol/mm³, 50 fmol/mm³, 60 fmol/mm³, 70 fmol/mm³, 80 fmol/mm³, 90 fmol/mm³, 100 fmol/mm³, 200 fmol/mm³, 300 fmol/mm³, 400 fmol/mm³, 500 fmol/mm³, 600 fmol/mm³, 700 fmol/mm³, 800 fmol/mm³, 900 fmol/mm³, or 1000 fmol/mm³. In certain embodiments, the forward and/or the reverse primers are immobilized to the solid surface at a density of about 200 fmol/mm³.

In some embodiments, the number of forward primers and the number of reverse primers are at a ratio of from about 1:20 to about 20:1, from about 1:15 to about 15:1, from about 1:12 to about 12:1, from about 1:9 to about 9:1, from about 1:5 to about 5:1, from about 1:2 to about 2:1, from about 1:1.5 to about 1.5:1, or from about 1:1.2 to about 1.2:1. In certain embodiments, the number of forward primers and the number of reverse primers are at a ratio of about 1:1.

In some embodiments, the 5' region of the forward primer and/or the 5' region of the reverse primer is 3-100 nucleotides (nt) in length, e.g., 5-100 nt, 10-100 nt, 20-100 nt, 30-100 nt, 40-100 nt, 50-100 nt, 3-90 nt, 3-80 nt, 3-70 nt, 3-60 nt, 3-50 nt, 3-40 nt, 3-30 nt, 3-20 nt, 5-90 nt, 8-80 nt, 10-70 nt, 15-60 nt, 18-50 nt, 20-45 nt, 20-40 nt, or 20-30 nt in length. In some embodiments, the 5' region of the forward primer and/or the 5' region of the reverse primer is 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 35 nt, 40 nt, 45 nt, 50 nt, 55 nt, 60 nt, 65 nt, 70 nt, 75 nt, 80 nt, 85 nt, 90 nt, 95 nt, or 100 nt in length. In some embodiments, the 5' region of the forward primer and the 5' region of the reverse primer have the same length. In some embodiments, the 5' region of the forward primer and the 5' region of the reverse primer have different lengths. For example, the 5' region of the forward primer is 20 nt in length and the 5' region of the reverse primer is 10 nt in length.

In some embodiments, the 5' region of the forward primer and the 5' region of the reverse primer are completely complementary to each other. In some embodiments, the 5' region of the forward primer and the 5' region of the reverse primer are partially complementary to each other with at least one mismatch. In some embodiments, the 5' region of the forward primer and the 5' region of the reverse primer are partially complementary to each other with one, two, or three mismatches.

In some embodiments, the 5' region of the forward primer and/or the 5' region of the reverse primer comprise one or more stretches of identical bases. In some embodiments, each of the one or more stretches comprises at least three identical bases (e.g., at least 3, 4, 5, 6, 7, 8, 9, or 10 identical bases). In some embodiments, the 5' region of the forward primer is a poly-A strand and the 5' region of the reverse primer is a poly-T strand. In some embodiments, the 5' region of the forward primer is a poly-T strand and the 5' region of the reverse primer is a poly-A strand.

In some embodiments, the 3' region of the forward primer and/or the 3' region of the reverse primer is 20-50 nucleotides (e.g., 20, 21, 22, 23, 24, 25, 26, 27, 28, 29, 30, 31, 32, 33, 34, 35, 36, 37, 38, 39, 40, 41, 42, 43, 44, 45, 46, 47 ,48, 49, or 50 nucleotides) in length. In some embodiments, the 3' region of the forward primer is 24 nucleotides in length. In some embodiments, the 3' region of the forward primer has a nucleotide sequence of 5'-CAAGCAGAAGACGGCATACGAGAT-3' (SEQ ID NO: 1). In some embodiments, the 3' region of the reverse primer is 29 nucleotides in length. In some embodiments, the 3' region of the reverse primer has a nucleotide sequence of 5'-AATGAUACGGCGACCACCGAGATCTACAC-3' (SEQ ID NO: 2). In some embodiments, the 5' region of the forward primer is 20 nucleotides in length and the 5' region of the reverse primer is 10 nucleotides in length. For example, the 5' region of the forward primer consists of 20 A and the 5' region of the reverse primer consists of 10 T, or the 5' region of the forward primer consists of 20 T and the 5' region of the reverse primer consists of 10 A. In certain embodiments, the forward primer has a nucleotide sequence of 5'-AAAAAAAAAAAAAAAAAAAACAAGCAGAAGACGGCATACGAGAT-3' (SEQ ID NO: 3); and the reverse primer has a nucleotide sequence of 5'-TTTTTTTTTTAATGAUACGGCGACCACCGAGATCTACAC-3' (SEQ ID NO: 4).

In some embodiments, the full length of the forward primer is about 15-150, about 20-120, about 25-100, about 30-80, about 30-60, about 35-55, about 35-50, or about 35-45 nucleotides. In some embodiments, the full length of the reverse primer is about 15-150, about 20-120, about 25-100, about 30-80, about 30-60, about 35-55, about 35-50, or about 35-45 nucleotides. In some embodiments, the immobilized forward primer has a Tₘ of 50-70°C, 52-68°C, 55-65°C, 58-64°C, 58-62 °C, or 58-60°C. In some embodiments, the immobilized reverse primer has a Tₘ of 50-70°C, 52-68°C, 55-65°C, 58-64°C, 60-64 °C, or 62-64°C. In certain embodiments, the Tₘ of the immobilized forward primer and/or the immobilized reverse primer is calculated by commercially available oligo analysis tools such as the Integrated DNA Technologies (IDT) tools under a setting of 50 mM Na⁺ and no Mg²⁺.

In some embodiments, the plurality of forward primers all have the same nucleotide sequence. In some embodiments, the plurality of reverse primers all have the same nucleotide sequence.

In some embodiments, the forward primers and/or the reverse primers are immobilized to the solid surface via their 5' ends. In some embodiments, the forward primers and/or the reverse primers are immobilized to the solid surface via their 3' ends. In some embodiments, the forward primers and/or the reverse primers are immobilized to the solid surface via an internal nucleotide.

In some embodiments, the forward primer and/or the reverse primer are covalently linked to the solid surface. One exemplary method that can be employed to immobilize the forward and/or reverse primers to the solid surface is the use of click chemistry-covered surfaces functionalized with azide, NHS, etc. For example, the forward primer and/or the reverse primer are designed with an appropriate modification on its 5' end, 3' end, or an internal nucleotide (e.g., DBCO for azide functionalized surfaces, or amine for NHS functionalized surfaces, etc.). The first step in such an exemplary method is applying the 5' end, 3' end, or internally modified forward primer and/or reverse primer on the functionalized surface to allow for the conjugation of the primers to the surface. The surface is then washed thoroughly to get rid of any unbound primers, and all of the unused functional groups are blocked for any non-specific binding. Additional methods for covalently linking the primers to the solid surface include but are not limited to, e.g., azide-alkyne; Amine-reactive -NH2 + NHS ester/ Imidoester /Pentafluorophenyl ester /Hydroxymethyl phosphine/ Epoxide/ Isocyanate; Carboxyl-to-amine reactive -COOH + Carbodiimide (e.g., EDC); Sulfhydryl-reactive -SH + Maleimide /Haloacetyl (bromo-, chloro-, or iodo-) /Pyridyl disulfide /Thiosulfonate /Vinyl sulfone; Aldehyde-reactive (e.g., oxidized sugars, carbonyls) -CHO+ Hydrazide /Alkoxyamine /NHS ester; Hydroxyl (nonaqueous)-reactive -OH+ Isocyanate; photoreactive cross linking( e.g., aryl azides+ nucleophile (e.g., primary amine), diazirine+ amino acid side chain or peptide backbone), etc.. In some embodiments, the primers are 5' DBCO-modified and the solid surface is azide functionalized surface. In some embodiments, the primers are 5' amine-modified and the solid surface is NHS functionalized surface.

In some embodiments, the solid surface is treated with polymers with azide groups. In some embodiments, the forward and/or the reverse primers are immobilized to the surface by modifying an internal base with an amine or alkyne. For example, in certain embodiments, the chemically modified reverse primer is 5'-TTTTTTTTT/i5OCTdU/TTTTTTTTAATGAUACGGCGACCACCGAGATCTACAC-3' (SEQ ID NO: 5). /i5OCTdU/ has a structure as follows:

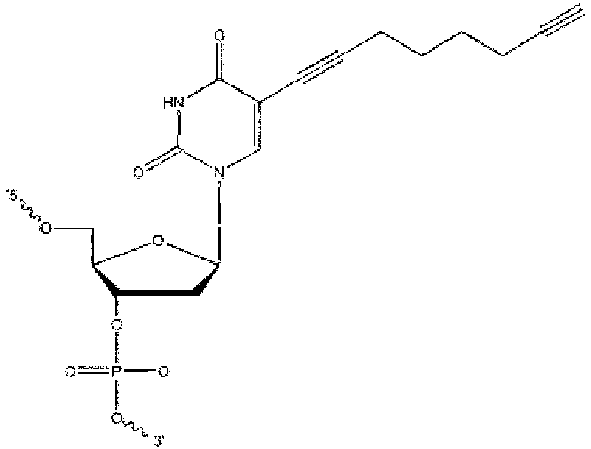

In certain embodiments, copper click reaction can be used to immobilize the forward and/or the reverse primer on a solid surface.

In some embodiments, the 3' end of the immobilized forward and/or reverse primers are free to elongate.

The surface can be any solid support. In some embodiments, the surface is the surface of a flow cell. In some embodiments, the surface is a slide, a chip (*e.g.*, the surface of a gene chip), a microwell plate, a plate, a tube, or a fluidic channel. In some embodiments, the surface is a bead (*e.g.*, a paramagnetic bead). A different example is the use of fluidics channels, which can be integrated into a machine that allows for washing steps. In some embodiments, the surface is an inner surface of the microfluidic devices described herein.

In some embodiments, the surface is a 3D polymer.

### Nucleic Acid Library Preparation

In some embodiments, the method further comprises preparing the nucleic acid library prior to step (b). In some embodiments, preparing the nucleic acid library comprises: (i) obtaining nucleic acids from a biological sample, (ii) fragmenting the nucleic acids to proper sizes, and (iii) adding adapters to both ends of the nucleic acid fragments such that the adapter at one end comprises a sequence that corresponds to or is complementary to the 3' region of the forward primer, and the adapter at the other end comprises a sequence that corresponds to or is complementary to the 3' region of the reverse primer.

The biological sample can be any cell, tissue, biopsy, plasma, blood, or other body fluid samples that contain nucleic acids (DNA or RNA). The biological sample can be obtained from a healthy subject or a patient. In some embodiments, the methods described herein further comprise a sample preparation step, for example, a nucleic acid purification step, a sample enrichment step, or a reverse transcription step for a RNA library.

In some embodiments, the sample is a purified nucleic acid (e.g., a purified DNA sample, a purified RNA sample, etc.) sample. In some embodiments, the sample is an unpurified sample, and the methods described herein include a nucleic acid purification step prior to contacting the sample to a solid surface. The sample can be purified using any known methods in the art for DNA or RNA purification, or using commercially available kits.

A variety of methods can be used to generate the nucleic acid (DNA, RNA, or cDNA) fragments, including but not limited to, physical methods (e.g., sonication), enzymatic methods (e.g., by using endonucleases such as DNase I, Fragmentase, etc.) or chemical methods. Fragmentation can also be done with commercial enzymatic kits.

The optimized size for nucleic acid fragments depends from the type of analysis. In some embodiments, the size of the nucleic acid fragments can be from 50 to 5,000 bp (e.g., 50-4500, 50-4000, 50-3500, 50-3000, 50-2500, 50-2000, 50-1500, 50-1200, 50-1000, 100-1000, 200-1000, 300-1000, 400-1000, 500-1000, 100-900, 200-800, 300-700, 400-700, 400-600, 400-500, 100-500, 100-400, 100-300, 200-500, 200-400, 200-300, etc.).

In some embodiments, the nucleic acid library comprises at least two nucleic acid molecules that have neither the same nor the complementary sequence. In some embodiments, the nucleic acid library comprises a plurality of nucleic acid molecules that have neither the same nor the complementary sequence. In some embodiments, the nucleic acid library is a DNA library. In some embodiments, the DNA library comprises single-strand DNA molecules. In some embodiments, the DNA library comprises double-strand DNA molecules. In some embodiments, the method further comprises denaturing the nucleic acid library prior to step (c) to generate single-strand polynucleotides. In some embodiments, the DNA library is a cDNA library or a genomic DNA library.

In some embodiments, the nucleic acid library is a RNA library (e.g., a total RNA sample, a mRNA sample, a microRNA sample, etc.). In some embodiments, reverse transcription is performed to generate the complementary DNA library (e.g., cDNA library) before contacting the library to the solid surface at step (b).

In some embodiments, the methods described herein can be used to generate clusters of amplicons for sequencing, detecting, and/or quantifying one or more nucleic acid libraries in a pooled manner. For example, in some embodiments, each nucleic acid library is generated with a unique oligonucleotide barcode, either from one end of the strand or from both ends of the strand, for example, by adding adapters that comprise a unique oligonucleotide barcode. Following that, pooled nucleic acid libraries can be applied to the solid surface on which a plurality of forward and/or reverse primers are immobilized. In some embodiments, the barcode is attached to one end of the nucleic acid, and spots hold one primer to complement and elongate the barcode side and one primer to complement the nucleic acid (to be detected) side. In some embodiments, the barcode is attached to both ends of nucleic acid, and spots hold primers complementing the barcodes fitting to amplify the stretch held between them.

In some embodiments, the method further comprises a step of removing fragments of undesired size and/or all adapters dimers.

### Amplification

In some embodiments, the amplification process is a polymerase chain reaction (PCR) or a recombinase polymerase amplification (RPA).

In some embodiments, step (c) comprises: (i) annealing the nucleic acid molecules from the library to the immobilized forward or reverse primers, (ii) generating immobilized DNA strands that are complementary to the nucleic acid molecules from the library by extending the forward or reverse primers, (iii) denaturing the double-strand nucleic acids, and (iv) washing away the nucleic acid molecules that are not immobilized to the solid surface. The annealing (i) and elongation (ii) steps can be considered as the seeding step. In some embodiments, denaturation (iii) and washing (iv) steps can be applied to ensure that only covalently bound material stays in the system and ends the seeding step. In some embodiments, the washing step is not applied and continuous seeding occurs.

In some embodiments, the starting material is RNA, and no denaturation takes place in sample preparation and the elongation step in the seeding stage is done using reverse transcriptase.

In some embodiments, step (c) further comprises one or more cycles of bridge PCR during which the immobilized DNA strand extended from the forward primer hybridizes with the reverse primer to generate the complementary strand, and/or the immobilized DNA strand extended from the reverse primer hybridizes with the forward primer to generate the complementary strand.

In some embodiments, the one or more cycles of bridge PCR generate: (1) an immobilized DNA strand extended from the immobilized forward primer which comprises, in 5' to 3' order, the 5' region of the forward primer, a sequence that corresponds to or is complementary to a nucleic acid molecule of the nucleic acid library, and a sequence that is complementary to the 5' region of the reverse primer or to the 3' portion of the 5' region of the reverse primer; and (2) an immobilized DNA strand extended from the immobilized reverse primer which comprises, in 5' to 3' order, the 5' region of the reverse primer, a sequence that corresponds to or is complementary to a nucleic acid molecule of the DNA library, and a sequence that is complementary to the 5' region of the forward primer or to the 3' portion of the 5' region of the forward primer. In some embodiments, rounds of bridge amplification (e.g., bPCR) can generate clusters of amplicons. Each cluster is seeded by (i.e., originated out of) a single nucleic acid molecule seed.

In some embodiments, step (c) further comprises a supersurface PCR during which 3' end of the immobilized DNA strand extended from the forward primer hybridizes with 3' end of the immobilized DNA strand extended from the reverse primer, and each of the two immobilized DNA strands extends at its 3' end using the other immobilized DNA strand as a template.

In some embodiments, step (c) comprises 5 to 75 cycles of PCR, or 15 to 50 cycles of PCR. For example, without limitation, step (c) may comprise 5, 10, 15, 20, 25, 30, 35, 40, 45, 50, 55, 60, 65, or 70 cycles of PCR. In some embodiments, the average cluster signal intensity is about 35-50 after 15 cycles of PCR.

In some embodiments, the method further comprises, after step (c): (d) incubating the solid surface with an exonuclease for a period of time; and (e) conducting a second phase of polymerase chain reaction (PCR) to generate the clusters of amplicons. In some embodiments, the exonuclease is selected from exonuclease I, exonuclease T, exonuclease P1, and exonuclease VII. In some embodiments, unused forward primers and reverse primers are removed from the solid surface by the exonuclease at step (d). In some embodiments, step (e) comprises a supersurface PCR during which 3' end of the immobilized DNA strand extended from the forward primer hybridizes with 3' end of the immobilized DNA strand extended from the reverse primer, and each of the two immobilized DNA strands extends at the 3' end using the other immobilized DNA strand as a template.

In some embodiments, step (e) comprises 5-50 cycles of PCR, e.g., 5, 10, 15, 20, 25, 30, 35, 40, 45, or 50 cycles of PCR. In some embodiments, step (e) comprises 25 cycles of PCR.

In some embodiments, step (c) and step (e) comprise a total of 30-60 cycles of PCR, e.g., 30, 35, 40, 45, 50, 55, or 60 cycles of PCR. In some embodiments, step (c) comprises 15 cycles of PCR and step (e) comprises 25 cycles of PCR. In some embodiments, the average cluster signal intensity is about 75-100 after completion of 15 cycles of PCR at step (c) and 25 cycles of PCR at step (e).

In some embodiments, each cluster of amplicons may contain from about 100 to about 10000 copies of amplicons after completion of 15 cycles of PCR at step (c) and 25 cycles of PCR at step (e). In some embodiments, the density of the clusters of amplicons ranges from about 300k/mm² to about 500k/mm² after completion of 15 cycles of PCR at step (c) and 25 cycles of PCR at step (e).

One of skill in the art would readily know that incubation temperature and time at step (c) need to be adjusted according to the sample complexity, the amplification method, and the primers Tₘ. These parameters can impact the sensitivity and the specificity of the reaction.

### Sequencing and Detection

In some embodiments, the method further comprises sequencing the clusters of amplicons to obtain sequence information for the nucleic acid library. Depending on the type of the nucleic acid library, the methods described herein can be used for genomic sequencing (e.g., whole-genome sequencing, exome sequencing, de novo sequencing, or targeted sequencing), transcriptomic sequencing (e.g., total RNA and mRNA sequencing, targeted RNA sequencing, or small RNA and noncoding RNA sequencing), or epigenomic sequence (e.g., methylation sequencing, ChIP sequencing, or ribosome profiling).

In some embodiments, the clusters of amplicons are sequenced using standard sequencing reagents and procedures known in the art. For example, the clusters of amplicons may be sequenced by synthesis by incorporating an intrinsically fluorescent or labeled nucleotide, e.g., an intrinsically fluorescent or labeled dUTP , dGTP, dCTP, dATP, etc., and the flow cell is imaged and the emission from each cluster is recorded for repeated cycles to generate a sequence read.

In some embodiments, the method further comprises hybridizing the clusters of amplicons with a probe to detect a target polynucleotide from the nucleic acid library.

In some embodiments, the clusters of amplicons are detected by a surface bound or suspended probe, e.g., a Taqman probe, molecular beacon, or scorpion, etc. In some embodiments, the surface bound probe is in the same cluster as the primer. In some embodiments, the surface bound probe is bound by the 3' end.

The probes or labeled dNTPs can be labeled with many different molecules for either immediate or for secondary reporting. For example, they can be labeled with biotin, DIG, and with either fluorescent, luminescent (e.g., dppz, Ruthenium (II) complex, etc.), colorimetric (e.g., gold, Crystal Violet, HRP+TMB, Alkaline phosphatase, palladium, platinum, magnetic nanoparticle, BSA-MnO2 NPs, graphene oxide, Carbon dots), or for electrochemical detection.

### Devices

In some aspects, provided herein is a microfluidic device comprising a solid surface immobilized with a plurality of forward primers and a plurality of reverse primers, wherein (i) each forward primer comprises, in 5' to 3' order, a 5' region and a 3' region; (ii) each reverse primer comprises, in 5' to 3' order, a 5' region and a 3' region, and (iii) the 5' region of the forward primer and the 5' region of the reverse primer are fully or partially complementary to each other. In some embodiments, the solid surface is a flow cell.

In some embodiments, the forward and/or the reverse primers are immobilized on a 3-dimensional surface. In some such embodiments, the forward and/or the reverse primers are immobilized to the solid surface at a density of about 1 fmol/mm² to about 1000 fmol/mm², for examples, from 10 fmol/mm² to about 1000 fmol/mm², 50 fmol/mm² to about 1000 fmol/mm², 100 fmol/mm² to about 1000 fmol/mm², 500 fmol/mm² to about 1000 fmol/mm², from 1 fmol/mm² to about 500 fmol/mm², 1 fmol/mm² to about 100 fmol/mm², 1 fmol/mm² to about 50 fmol/mm², or 1 fmol/mm² to about 10 fmol/mm². In some embodiments, the forward and/or the reverse primers are immobilized to the solid surface at a density of about 1 fmol/mm², 10 fmol/mm², 20 fmol/mm², 30 fmol/mm², 40 fmol/mm², 50 fmol/mm², 60 fmol/mm², 70 fmol/mm², 80 fmol/mm², 90 fmol/mm², 100 fmol/mm², 200 fmol/mm², 300 fmol/mm², 400 fmol/mm², 500 fmol/mm², 600 fmol/mm², 700 fmol/mm², 800 fmol/mm², 900 fmol/mm², or 1000 fmol/mm². In certain embodiments, the forward and/or the reverse primers are immobilized to the solid surface at a density of about 200 fmol/mm².

In some embodiments, the forward and/or the reverse primers are immobilized on a 3-dimensional surface. In some such embodiments, the forward and/or the reverse primers are immobilized to the solid surface at a density of about 1 fmol/mm³ to about 1000 fmol/mm³, for examples, from 10 fmol/mm³ to about 1000 fmol/mm³, 50 fmol/mm³ to about 1000 fmol/mm³, 100 fmol/mm³ to about 1000 fmol/mm³, 500 fmol/mm³ to about 1000 fmol/mm³, from 1 fmol/mm³ to about 500 fmol/mm³, 1 fmol/mm³ to about 100 fmol/mm³, 1 fmol/mm³ to about 50 fmol/mm³, or 1 fmol/mm³ to about 10 fmol/mm³. In some embodiments, the forward and/or the reverse primers are immobilized to the solid surface at a density of about 1 fmol/mm³, 10 fmol/mm³, 20 fmol/mm³, 30 fmol/mm³, 40 fmol/mm³, 50 fmol/mm³, 60 fmol/mm³, 70 fmol/mm³, 80 fmol/mm³, 90 fmol/mm³, 100 fmol/mm³, 200 fmol/mm³, 300 fmol/mm³, 400 fmol/mm³, 500 fmol/mm³, 600 fmol/mm³, 700 fmol/mm³, 800 fmol/mm³, 900 fmol/mm³, or 1000 fmol/mm³. In certain embodiments, the forward and/or the reverse primers are immobilized to the solid surface at a density of about 200 fmol/mm³.

In some embodiments, the number of forward primers and the number of reverse primers are at a ratio of from about 1:20 to about 20:1, from about 1:15 to about 15:1, from about 1:12 to about 12:1, from about 1:9 to about 9:1, from about 1:5 to about 5:1, from about 1:2 to about 2:1, from about 1:1.5 to about 1.5:1, or from about 1:1.2 to about 1.2:1. In certain embodiments, the number of forward primers and the number of reverse primers are at a ratio of about 1:1.

In some embodiments, the 5' region of the forward primer and/or the 5' region of the reverse primer is 3-100 nucleotides (nt) in length, e.g., 5-100 nt, 10-100 nt, 20-100 nt, 30-100 nt, 40-100 nt, 50-100 nt, 3-90 nt, 3-80 nt, 3-70 nt, 3-60 nt, 3-50 nt, 3-40 nt, 3-30 nt, 3-20 nt, 5-90 nt, 8-80 nt, 10-70 nt, 15-60 nt, 18-50 nt, 20-45 nt, 20-40 nt, or 20-30 nt in length. In some embodiments, the 5' region of the forward primer and/or the 5' region of the reverse primer is 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 35 nt, 40 nt, 45 nt, 50 nt, 55 nt, 60 nt, 65 nt, 70 nt, 75 nt, 80 nt, 85 nt, 90 nt, 95 nt, or 100 nt in length. In some embodiments, the 5' region of the forward primer and the 5' region of the reverse primer have the same length. In some embodiments, the 5' region of the forward primer and the 5' region of the reverse primer have different lengths. For example, the 5' region of the forward primer is 20 nt in length and the 5' region of the reverse primer is 10 nt in length.

In some embodiments, the 5' region of the forward primer and the 5' region of the reverse primer are completely complementary to each other. In some embodiments, the 5' region of the forward primer and the 5' region of the reverse primer are partially complementary to each other with at least one mismatch. In some embodiments, the 5' region of the forward primer and the 5' region of the reverse primer are partially complementary to each other with one, two, or three mismatches.

In some embodiments, the 5' region of the forward primer and/or the 5' region of the reverse primer comprise one or more stretches of identical bases. In some embodiments, each of the one or more stretches comprises at least three identical bases (e.g., at least 3, 4, 5, 6, 7, 8, 9, or 10 identical bases). In some embodiments, the 5' region of the forward primer is a poly-A strand and the 5' region of the reverse primer is a poly-T strand. In some embodiments, the 5' region of the forward primer is a poly-T strand and the 5' region of the reverse primer is a poly-A strand.

In some embodiments, the 3' region of the forward primer and/or the 3' region of the reverse primer is 20-50 nucleotides (e.g., 20, 21, 22, 23, 24, 25, 26, 27, 28, 29, 30, 31, 32, 33, 34, 35, 36, 37, 38, 39, 40, 41, 42, 43, 44, 45, 46, 47 ,48, 49, or 50 nucleotides) in length. In some embodiments, the 3' region of the forward primer is 24 nucleotides in length. In some embodiments, the 3' region of the forward primer is 5'-CAAGCAGAAGACGGCATACGAGAT-3' (SEQ ID NO: 1). In some embodiments, the 3' region of the reverse primer is 29 nucleotides in length. In some embodiments, the 3' region of the reverse primer is 5'-AATGAUACGGCGACCACCGAGATCTACAC-3' (SEQ ID NO: 2). In some embodiments, the 5' region of the forward primer is 20 nucleotides in length and the 5' region of the reverse primer is 10 nucleotides in length. For example, the 5' region of the forward primer consists of 20 A and the 5' region of the reverse primer consists of 10 T, or the 5' region of the forward primer consists of 20 T and the 5' region of the reverse primer consists of 10 A. In certain embodiments, the forward primer 5'-AAAAAAAAAAAAAAAAAAAACAAGCAGAAGACGGCATACGAGAT-3' (SEQ ID NO: 3); and the reverse primer is 5'-TTTTTTTTTTAATGAUACGGCGACCACCGAGATCTACAC-3' (SEQ ID NO: 4).

In some embodiments, the full length of the forward primer is about 15-150, about 20-120, about 25-100, about 30-80, about 30-60, about 35-55, about 35-50, or about 35-45 nucleotides. In some embodiments, the full length of the reverse primer is about 15-150, about 20-120, about 25-100, about 30-80, about 30-60, about 35-55, about 35-50, or about 35-45 nucleotides. In some embodiments, the immobilized forward primer has a Tₘ of 50-70°C, 52-68°C, 55-65°C, 58-64 °C, 58-62 °C, or 58-60°C. In some embodiments, the immobilized reverse primer has a Tₘ of 50-70°C, 52-68°C, 55-65°C, 58-64°C, 60-64 °C, or 62-64°C. In certain embodiments, the Tₘ of the immobilized forward primer and/or the immobilized reverse primer is calculated by commercially available oligo analysis tools such as the Integrated DNA Technologies (IDT) tools under a setting of 50 mM Na⁺ and no Mg²⁺.

In some embodiments, the plurality of forward primers all have the same nucleotide sequence. In some embodiments, the plurality of reverse primers all have the same nucleotide sequence.

The exemplary methods of immobilizing the forward primers and/or the reverse primers to a solid surface described in the section above can be used to generate the microfluidic devices comprising a solid surface immobilized with a plurality of forward primers and a plurality of reverse primers as describe herein.

In some aspects, provided herein is a method of producing a microfluidic device described herein.

In some aspects, provided herein is a method of using a microfluidic device described herein to generate clusters of amplicons for a nucleic acid library. In some embodiments, the method comprises: (a) contacting the nucleic acid library with the solid surface of the microfluidic device described herein; and (b) performing an amplification process on the solid surface of the microfluidic device to generate the clusters of amplicons, whereby each nucleic acid molecule from the nucleic acid library is amplified into a clonal cluster of amplicons.

In some aspects, provided herein is a method of using a microfluidic device described herein to sequence a nucleic acid sample. In some embodiments, the method comprises: (a) preparing a nucleic acid library from the nucleic acid sample; (b) contacting the nucleic acid library with the solid surface of the microfluidic device described herein; (c) performing an amplification process on the solid surface of the microfluidic device to generate the clusters of amplicons, whereby each nucleic acid molecule from the nucleic acid library is amplified into a clonal cluster of amplicons; and (d) sequencing the clusters of amplicons to obtain sequence information for the nucleic acid sample.

In some aspects, provided herein is a method of using a microfluidic device described herein to detect a target polynucleotide from a nucleic acid sample. In some embodiments, the method comprises: (a) preparing a nucleic acid library from the nucleic acid sample; (b) contacting the nucleic acid library with the solid surface of the microfluidic device described herein; (c) performing an amplification process on the solid surface of the microfluidic device to generate the clusters of amplicons, whereby each nucleic acid molecule from the nucleic acid library is amplified into a clonal cluster of amplicons; and (d) hybridizing the clusters of amplicons with a probe to detect a target polynucleotide from the nucleic acid sample.

The exemplary methods for preparing the nucleic acid library, the amplification process, and the sequencing and detection methods described in the section above can be applied to the methods of using a microfluidic device as described herein.

### EXAMPLES

### Example 1

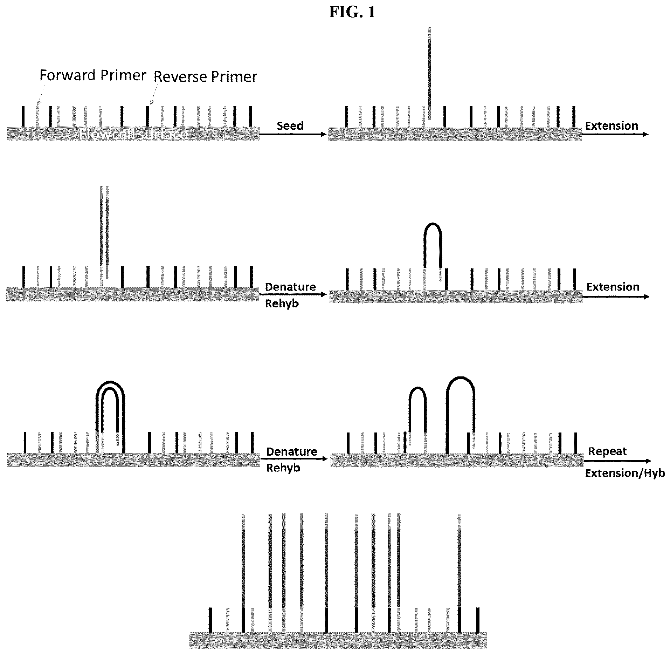

Bridge surface amplification (bPCR) has been used to generate DNA clusters for high-throughput sequencing. The workflow for a bPCR is illustrated in FIG. 1. As shown in FIG. 1, the surface is modified or grafted with forward and reverse primers which are complementary with DNA library primers. The library DNA is denatured and contacted with the surface primers. The hybridized DNA library is preserved after a washing step. The complementary stands immobilized on the surface are then synthesized by a polymerase. The hybridized strands are washed away after denaturing and washing steps. The other end of the newly synthesized strand contains the primer that is complementary to the surface primers, and the strand bends down to form a bridge-like structure. Another complementary strand is then synthesized. After a denaturing step, these reactions are repeated from 1 to 50 cycles to amplify these ssDNA. More than thousands of copies per cluster can be easily generated which provides a strong fluorescent signal for DNA sequencing. However, the more cycles of amplification, the larger the diameters of the clusters become. This limits the densities of the clusters on the surface that can be achieved since the clusters would be connected to each other after extra cycles of PCR reactions. To achieve a higher cluster density for a high throughput sequencing platform, smaller and dimmer clusters have been employed with a high end optical design and an image sensor which are very expensive.

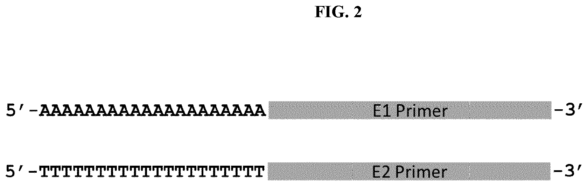

New methods were developed herein to amplify single-strand DNA (ssDNA) immobilized on a solid surface for DNA sequencing-related applications. To increase the copies of DNA in each cluster, new primers with extended tails were designed as shown in FIG.2. The forward primer E1 had an extended tail (polyA) at the 5' end of the primer. The reverse primer E2 had a complementary tail to E1's tail added to the 5' end of the primer. In this case, polyA and polyT were chosen. However, any complementary sequences can be used in this design including, e.g., CAT/GTA, AAT/TTA, or ATT/TAA. The length of the tail can be anywhere from 3 nucleotides to up to 100 nucleotides.

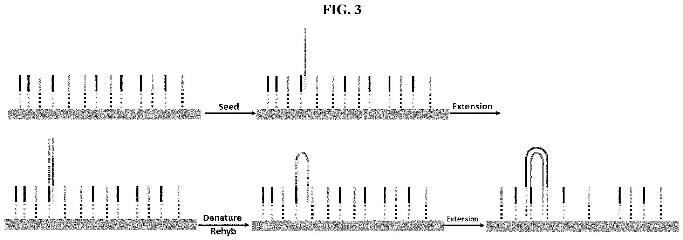

The seeding step and the first extension of supersurface amplification (ssPCR) are demonstrated in FIG. 3, which are very similar to the bridge amplification shown in FIG.1. However, during the first cycle of PCR amplification step, the newly synthesized strand extended beyond the E1' region (E1' refers to the region that is complementary to the E1 region), and a poly-T tail was also synthesized based on the polyA-E1 surface primers. The original strand also extended and a new polyA-E2' end (E2' refers to the region that is complementary to the E2 region) was synthesized.

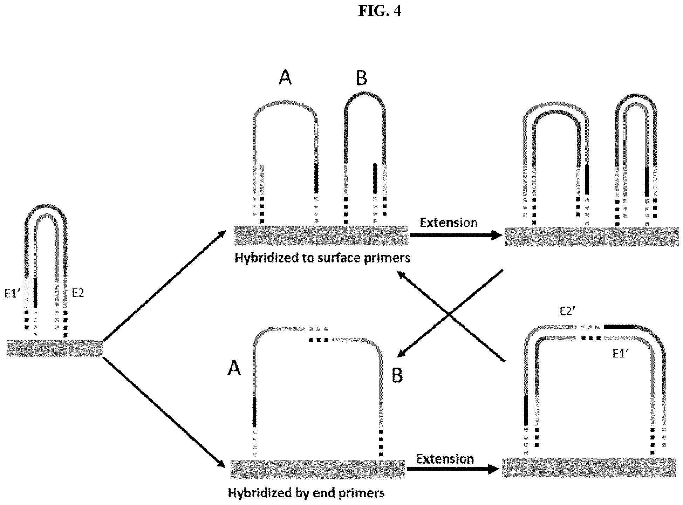

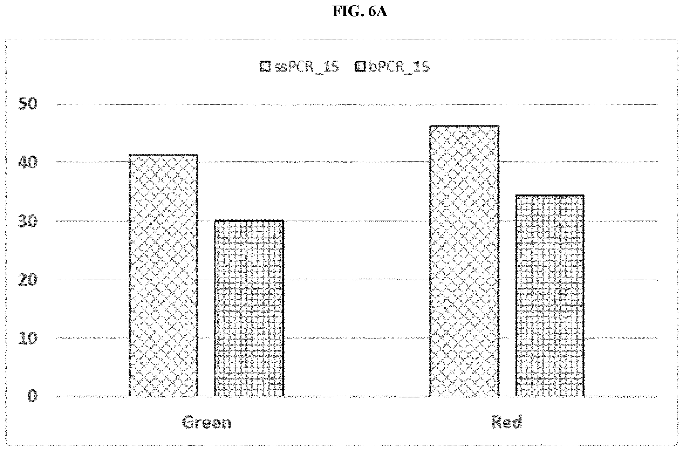

As shown in FIG. 4, after denaturing and rehybridization, the surface immobilized copies of DNA can hybridize to surface primers E1 and E2 and produce one copy of new DNA strand as shown on the top of FIG. 4. Alternatively, the newly synthesized polyA and polyT tail can hybridize each other, and one copy of single strand DNA (ssDNA) can be synthesized, as shown on the bottom of FIG. 4. Theoretically, the amplification efficiency of these two ways should be similar and the ideal number of copies after n cycles are both 2ⁿ. However, the first path consumes one surface primer for every duplication, and each immobilized primer is extended to generate a single copy of amplicon. The second path does not consume any surface primer since it produces the forward and reverse primer in every cycle, and multiple copies of amplicons can be generated from each immobilized primer. Thus, due to the limited supply of surface primers, the amplification efficiency of supersurface PCR was in fact improved compared to the regular bridge amplification. FIG. 6A shows average cluster signal intensities from green and red fluorescent channels for 15 cycles of ssPCR and bPCR. The data were analyzed by finding the maximum point of each clusters and plotted in two dimensional (image 1 as X axis and image 2 as y axis). Four clouds of clusters should be observed and the average maximum brightness was calculated for each type of clouds (A, T, C and G). As shown in FIG. 6A, the average cluster intensities of ssPCR with 15 cycles in both green and red fluorescent channels were about 35% higher than the regular bPCR with 15 cycles of amplification reactions.

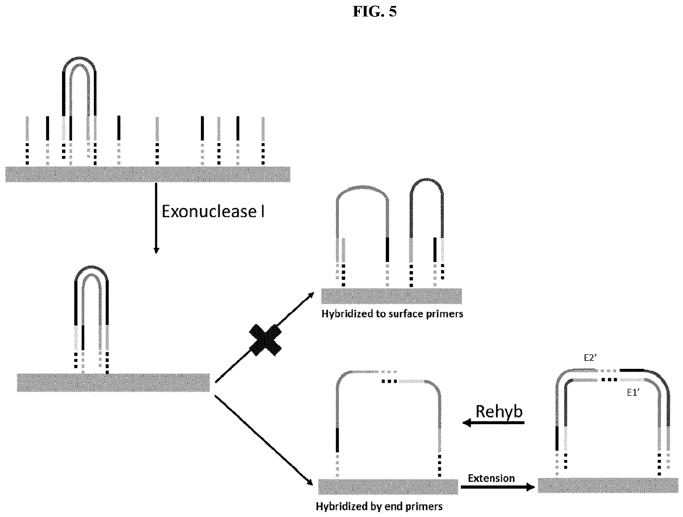

To boost the cluster intensity and limit the size of the cluster on the solid surface, the following treatment was conducted. The ssPCR reaction was performed for 15 cycles initially, during which the surface immobilized DNA were amplified using both the first path (i.e., hybridizing to surface primers) and the second path (i.e., hybridizing by end primers) as described in FIG. 4. This initial amplification determined the sizes of clusters. Then the surface was digested with exonuclease I to remove all single-strand DNAs (ssDNAs) on the surface which were mainly unused surface primers as illustrated in FIG. 5. Another 25 cycles of ssPCR reaction were employed to duplicated DNA strands using the second path only, as shown in FIG. 5. The first path, hybridizing the synthesized strands to the surface E1 or E2 primers, can't be achieved since the primers had been digested by enzyme exonuclease I. With this method, the DNA clusters can grow vertically but not horizontally on the solid surface, thereby limiting the diameter while maintaining a high signal intensity of each clusters on the solid surface.

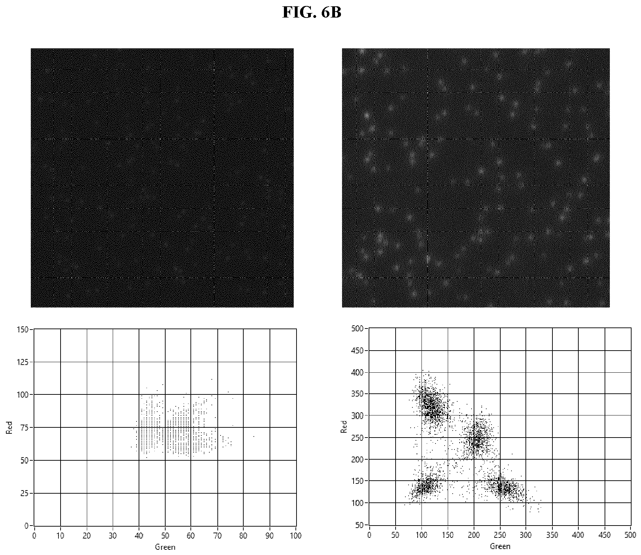

As shown in FIG. 6B, the clusters were barely detectable for bPCR with the traditional primer design and a combination of the initial 15 cycles PCR and the additional 25 cycles after the enzyme treatment. In contrast, the clusters were both bright and small for ssPCR with the new primer design shown in FIG. 2 using the same PCR protocol. The four clouds of A, T, G, C clusters plotted in FIG. 6B also clearly demonstrated the separation between A, T, G, C clouds, which was perfect to base calling algorithms. In contrast, bPCR's four clouds barely showed the differences between the A, T, G, C clouds.

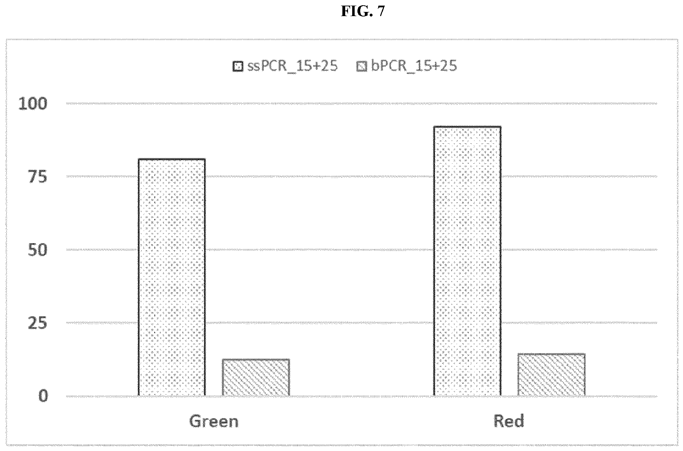

The average intensities of clusters in green and red channels are shown in FIG. 7. The average intensities of the clusters generated by ssPCR are about 5.4 times more than those of the clusters generated by the regular bPCR with similar amplification reaction conditions.

Due to the high signal intensities and the small diameters of the clusters generated by ssPCR, the density of the clusters was greatly increased by ssPCR. For example, the cluster density for Nextseq, an illumina sequencing platform using bridge amplification, is typically from 150k/mm² to 300k/mm². A density from about 300k/mm² to about 500k/mm² with an intensity that is comparable to Nextseq can be achieved by ssPCR in a similar format to Nextseq. Each cluster from the ssPCR reaction can contain from about 100 copies to about 10000 copies of amplicons.

### Incorporation by Reference

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

### Equivalents

Those skilled in the art will recognize, or be able to ascertain using no more than routine experimentation, many equivalents to the specific embodiments of the invention described herein. Such equivalents are intended to be encompassed by the following claims.

### Embodiments

1. A method of generating clusters of amplicons for a nucleic acid library, comprising:
   (a) providing a solid surface immobilized with a plurality of forward primers and a plurality of reverse primers, wherein (i) each forward primer comprises, in 5' to 3' order, a 5' region and a 3' region; (ii) each reverse primer comprises, in 5' to 3' order, a 5' region and a 3' region; and (iii) the 5' region of the forward primer and the 5' region of the reverse primer are fully or partially complementary to each other;
   (b) contacting the nucleic acid library with the solid surface, wherein each nucleic acid molecule of the library comprises, in 5' to 3' order: (i) a first adapter comprising a sequence that corresponds to the sequence of the 3' region of the reverse primer, a template sequence, and a second adapter comprising a sequence that is complementary to the 3' region of the forward primer; or (ii) a first adapter comprising a sequence that corresponds to the sequence of the 3' region of the forward primer, a template sequence, and a second adapter comprising a sequence that is complementary to the 3' region of the reverse primer; and
   (c) performing an amplification process on the solid surface to generate the clusters of amplicons, whereby each nucleic acid molecule from the nucleic acid library is amplified into a clonal cluster of amplicons.
2. The method of embodiment 1, wherein the method further comprises immobilizing the plurality of forward primers and the plurality of reverse primers to the solid surface prior to step (a).
3. The method of embodiment 1 or 2, wherein the forward and/or reverse primers are immobilized to the solid surface at a density of about 1 fmol/mm² to about 1000 fmol/mm², and optionally wherein the forward and/or reverse primers are immobilized to the solid surface at a density of about 200 fmol/mm².
4. The method of any one of embodiments 1-3, wherein the solid surface is a flow cell, a solid bead, a glass slide, or a multi-well plate.
5. The method of any one of embodiments 1-4, wherein the 5' region of the forward primer is 3-100 nucleotides in length.
6. The method of any one of embodiments 1-5, wherein the 5' region of the reverse primer is 3-100 nucleotides in length.
7. The method of any one of embodiments 1-6, wherein the 5' region of the forward primer and the 5' region of the reverse primer have the same length.
8. The method of any one of embodiments 1-7, wherein the 5' region of the forward primer and the 5' region of the reverse primer have different lengths.
9. The method of any one of embodiments 1-8, wherein the 5' region of the forward primer and the 5' region of the reverse primer are completely complementary to each other.
10. The method of any one of embodiments 1-8, wherein the 5' region of the forward primer and the 5' region of the reverse primer are partially complementary to each other with at least one mismatch.
11. The method of embodiment 10, wherein the 5' region of the forward primer and the 5' region of the reverse primer are partially complementary to each other with one, two, or three mismatches.
12. The method of any one of embodiments 1-11, wherein the 5' region of the forward primer and/or the 5' region of the reverse primer comprise one or more stretches of identical bases.
13. The method of any one of embodiments 1-12, wherein each of the one or more stretches comprises at least three identical bases.
14. The method of any one of embodiments 1-13, wherein the 5' region of the forward primer is a poly-A strand and the 5' region of the reverse primer is a poly-T strand, or the 5' region of the forward primer is a poly-T strand and the 5' region of the reverse primer is a poly-A strand.
15. The method of any one of embodiments 1-14, wherein the 3' region of the forward primer is 20-50 nucleotides in length.
16. The method of embodiment 15, wherein the 3' region of the forward primer is 24 nucleotides in length.
17. The method of embodiment 16, wherein the 3' region of the forward primer has a nucleotide sequence of 5'-CAAGCAGAAGACGGCATACGAGAT-3' (SEQ ID NO: 1).
18. The method of any one of embodiments 1-17, wherein the 3' region of the reverse primer is 20-50 nucleotides in length.
19. The method of embodiment 18, wherein the 3' region of the reverse primer is 29 nucleotides in length.
20. The method of embodiment 19, wherein the 3' region of the reverse primer has a nucleotide sequence of 5'-AATGAUACGGCGACCACCGAGATCTACAC-3' (SEQ ID NO: 2).
21. The method of any one of embodiments 15-20, wherein the 5' region of the forward primer is 20 nucleotides in length and the 5' region of the reverse primer is 10 nucleotides in length.
22. The method of embodiment 21, wherein (1) the 5' region of the forward primer consists of 20 A and the 5' region of the reverse primer consists of 10 T, or (2) the 5' region of the forward primer consists of 20 T and the 5' region of the reverse primer consists of 10 A.
23. The method of embodiment 22, wherein the forward primer has a nucleotide sequence of 5'- AAAAAAAAAAAAAAAAAAAACAAGCAGAAGACGGCATACGAGAT-3' (SEQ ID NO: 3); and the reverse primer has a nucleotide sequence of 5'-TTTTTTTTTTAATGAUACGGCGACCACCGAGATCTACAC-3' (SEQ ID NO: 4).
24. The method of any one of embodiments 1-23, wherein the plurality of forward primers all have the same nucleotide sequence.
25. The method of any one of embodiments 1-24, wherein the plurality of reverse primers all have the same nucleotide sequence.
26. The method of any one of embodiments 1-25, wherein the plurality of forward primers and the plurality of reverse primers are immobilized to the solid surface adjacent to each other.
27. The method of any one of embodiments 1-26, wherein the number of forward primers and the number of reverse primers are at a ratio of from about 1:20 to about 20:1, from about 1:9 to about 9:1, or from about 1:2 to about 2: 1.
28. The method of any one of embodiments 1-27, wherein the number of forward primers and the number of reverse primers are at a ratio of about 1:1.
29. The method of any one of embodiments 1-28, wherein the method further comprises preparing the nucleic acid library prior to step (b).
30. The method of embodiment 29, wherein preparing the nucleic acid library comprises: (i) obtaining nucleic acids from a biological sample, (ii) fragmenting the nucleic acids to proper sizes, and (iii) adding adapters to both ends of the nucleic acid fragments such that the adapter at one end comprises a sequence that corresponds to or is complementary to the 3' region of the forward primer, and the adapter at the other end comprises a sequence that corresponds to is complementary to the 3' region of the reverse primer.
31. The method of any one of embodiments 1-30, wherein the nucleic acid library comprises at least two nucleic acid molecules that have neither the same nor the complementary sequence.
32. The method of any one of embodiments 1-31, wherein the nucleic acid library is a DNA library.
33. The method of any one of embodiments 1-32, wherein the DNA library comprises single-strand DNA molecules.
34. The method of any one of embodiments 1-32, wherein the DNA library comprises double-strand DNA molecules.
35. The method of any one of embodiments 1-34, wherein the DNA library is a cDNA library or a genomic DNA library.
36. The method of any one of embodiments 1-35, wherein the amplification process is a polymerase chain reaction (PCR) or a recombinase polymerase amplification (RPA).
37. The method of any one of embodiments 1-36, wherein the method further comprises denaturing the nucleic acid library prior to step (c).
38. The method of any one of embodiments 1-37, wherein step (c) comprises: (i) annealing the nucleic acid molecules from the library to the immobilized forward or reverse primers, (ii) generating immobilized DNA strands that are complementary to the nucleic acid molecules from the library by extending the forward or reverse primers, (iii) denaturing the double-strand nucleic acids, and (iv) washing away the nucleic acid molecules that are not immobilized to the solid surface.
39. The method of any one of embodiments 1-38, wherein step (c) further comprises one or more cycles of bridge PCR during which the immobilized DNA strand extended from the forward primer hybridizes with the reverse primer to generate the complementary strand, and/or the immobilized DNA strand extended from the reverse primer hybridizes with the forward primer to generate the complementary strand.
40. The method of any one of embodiments 1-39, wherein the one or more cycles of bridge PCR generate: (1) an immobilized DNA strand extended from the immobilized forward primer which comprises, in 5' to 3' order, the 5' region of the forward primer, a sequence that corresponds to or is complementary to a nucleic acid molecule of the nucleic acid library, and a sequence that is complementary to the 5' region of the reverse primer or to the 3' portion of the 5' region of the reverse primer; and (2) an immobilized DNA strand extended from the immobilized reverse primer which comprises, in 5' to 3' order, the 5' region of the reverse primer, a sequence that corresponds to or is complementary to a nucleic acid molecule of the DNA library, and a sequence that is complementary to the 5' region of the forward primer or to the 3' portion of the 5' region of the forward primer.
41. The method of any one of embodiments 1-40, wherein step (c) further comprises a supersurface PCR during which 3' end of the immobilized DNA strand extended from the forward primer hybridizes with 3' end of the immobilized DNA strand extended from the reverse primer, and each of the two immobilized DNA strands extends at its 3' end using the other immobilized DNA strand as a template.
42. The method of any one of embodiments 1-41, wherein step (c) comprises 5 to 75 cycles of PCR.
43. The method of any one of embodiments 1-42, wherein step (c) comprises 15 to 50 cycles of PCR.
44. The method of embodiment 43, wherein the average cluster signal intensity is about 35-50 after 15 cycles of PCR.
45. The method of any one of embodiments 1-44, further comprising, after step (c):
   (d) incubating the solid surface with an exonuclease for a period of time; and
   (e) conducting a second phase of polymerase chain reaction (PCR) to generate the clusters of amplicons.
46. The method of embodiment 45, wherein the exonuclease is selected from exonuclease I, exonuclease T, exonuclease P1, and exonuclease VII.
47. The method of embodiments 45 or 46, wherein unused forward primers and reverse primers are removed from the solid surface by the exonuclease at step (d).
48. The method of any one of embodiments 45-47, wherein step (e) comprises a supersurface PCR during which 3' end of the immobilized DNA strand extended from the forward primer hybridizes with 3' end of the immobilized DNA strand extended from the reverse primer, and each of the two immobilized DNA strands extends at the 3' end using the other immobilized DNA strand as a template.
49. The method of any one of embodiments 45-48, wherein step (e) comprises 5-50 cycles of PCR.
50. The method of any one of embodiments 45-49, wherein step (e) comprises 25 cycles of PCR.
51. The method of any one of embodiments 45-50, wherein step (c) and step (e) comprise a total of 30-60 cycles of PCR.
52. The method of any one of embodiments 45-51, wherein step (c) comprises 15 cycles of PCR and step (e) comprises 25 cycles of PCR.
53. The method of embodiment 52, wherein the average cluster signal intensity is about 75-100 after 15 cycles of PCR at step (c) and 25 cycles of PCR at step (e).
54. The method of any one of embodiments 1-53, wherein the method further comprises sequencing the clusters of amplicons to obtain sequence information for the nucleic acid library.
55. The method of any one of embodiments 1-53, wherein the method further comprises hybridizing the clusters of amplicons with a probe to detect a target polynucleotide from the nucleic acid library.
56. A method of sequencing a nucleic acid sample comprising:
   (a) preparing a nucleic acid library from the nucleic acid sample;
   (b) generating clusters of amplicons using methods of any one of embodiments 1-53;
   (c) sequencing the clusters of amplicons to obtain sequence information for the nucleic acid sample.
57. A method of detecting a target polynucleotide from a nucleic acid sample comprising:
   (a) preparing a nucleic acid library from the nucleic acid sample;
   (b) generating clusters of amplicons using methods of any one of embodiments 1-53;
   (c) hybridizing the clusters of amplicons with a probe to detect a target polynucleotide from the nucleic acid sample.
58. The method of embodiment 56 or 57, wherein the nucleic acid sample is a total RNA sample or an mRNA sample, and step (a) comprises preparing a cDNA library from the total RNA or the mRNA sample.
59. A microfluidic device comprising a solid surface immobilized with a plurality of forward primers and a plurality of reverse primers, wherein (i) each forward primer comprises, in 5' to 3' order, a 5' region and a 3' region; (ii) each reverse primer comprises, in 5' to 3' order, a 5' region and a 3' region, and (iii) the 5' region of the forward primer and the 5' region of the reverse primer are fully or partially complementary to each other.
60. The microfluidic device of embodiment 59, wherein the solid surface is a flow cell.
61. The microfluidic device of embodiments 59 or 60, wherein the 5' region of the forward primer is 3-100 nucleotides in length.
62. The microfluidic device of any one of embodiments 59-61, wherein the 5' region of the reverse primer is 3-100 nucleotides in length.
63. The microfluidic device of any one of embodiments 59-62, wherein the 5' region of the forward primer and the 5' region of the reverse primer have the same length.
64. The microfluidic device of any one of embodiments 59-63, wherein the 5' region of the forward primer and the 5' region of the reverse primer have different lengths.
65. The microfluidic device of any one of embodiments 59-64, wherein the 5' region of the forward primer and the 5' region of the reverse primer are completely complementary to each other.
66. The microfluidic device of any one of embodiments 59-65, wherein the 5' region of the forward primer and the 5' region of the reverse primer are partially complementary to each other with at least one mismatch.
67. The microfluidic device of embodiment 66, wherein the 5' region of the forward primer and the 5' region of the reverse primer are partially complementary to each other with one, two, or three mismatches.
68. The microfluidic device of any one of embodiments 59-67, wherein the 5' region of the forward primer and/or the 5' region of the reverse primer comprise one or more stretches of identical bases.
69. The microfluidic device of any one of embodiments 59-68, wherein each of the one or more stretches comprises at least three identical bases.
70. The microfluidic device of any one of embodiments 59-69, wherein (1) the 5' region of the forward primer is a poly-A strand and the 5' region of the reverse primer is a poly-T strand, or (2) the 5' region of the forward primer is a poly-T strand and the 5' region of the reverse primer is a poly-A strand.
71. The microfluidic device of any one of embodiments 59-70, wherein the 3' region of the forward primer is 20-50 nucleotides in length.
72. The microfluidic device of embodiment 71, wherein the 3' region of the forward primer is 24 nucleotides in length.
73. The microfluidic device of embodiment 72, wherein the 3' region of the forward primer has a nucleotide sequence of 5'-CAAGCAGAAGACGGCATACGAGAT-3' (SEQ ID NO: 1).
74. The microfluidic device of any one of embodiments 59-73, wherein the 3' region of the reverse primer is 20-50 nucleotides in length.
75. The microfluidic device of embodiment 74, wherein the 3' region of the reverse primer is 29 nucleotides in length.
76. The microfluidic device of embodiment 75, wherein the 3' region of the reverse primer has a nucleotide sequence of 5'-AATGAUACGGCGACCACCGAGATCTACAC-3' (SEQ ID NO: 2).
77. The microfluidic device of any one of embodiments 71-76, wherein the 5' region of the forward primer is 20 nucleotides in length and the 5' region of the reverse primer is 10 nucleotides in length.
78. The microfluidic device of embodiment 77, wherein (1) the 5' region of the forward primer consists of 20 A and the 5' region of the reverse primer consists of 10 T, or (2) the 5' region of the forward primer consists of 20 T and the 5' region of the reverse primer consists of 10 A.
79. The microfluidic device of embodiment 78, wherein the forward primer has a nucleotide sequence of 5'-AAAAAAAAAAAAAAAAAAAACAAGCAGAAGACGGCATACGAGAT-3' (SEQ ID NO: 3); and the reverse primer has a nucleotide sequence of 5'-TTTTTTTTTTAATGAUACGGCGACCACCGAGATCTACAC-3' (SEQ ID NO: 4).
80. The microfluidic device of any one of embodiments 59-79, wherein the plurality of forward primers all have the same nucleotide sequence.
81. The microfluidic device of any one of embodiments 59-80, wherein the plurality of reverse primers all have the same nucleotide sequence.
82. The microfluidic device of any one of embodiments 59-81, wherein the plurality of forward primers and the plurality of reverse primers are immobilized to the solid surface adjacent to each other.
83. The microfluidic device of any one of embodiments 59-82, wherein the number of forward primers and the number of reverse primers are at a ratio of from about 1:20 to about 20:1, from about 1:9 to about 9:1, or from about 1:2 to about 2:1.
84. The microfluidic device of any one of embodiments 59-83, wherein the number of forward primers and the number of reverse primers are at a ratio of about 1:1.
85. The microfluidic device of any one of embodiments 59-84, wherein the forward and/or reverse primers are immobilized to the solid surface at a density of about 1 fmol/mm² to about 1000 fmol/mm².
86. The microfluidic device of embodiment 85, wherein the forward and/or reverse primers are immobilized to the solid surface at a density of about 200 fmol/mm².
87. A method of producing a microfluidic device of any one of embodiments 59-86.
88. A method of using a microfluidic device of any one of embodiments 59-86 to generate clusters of amplicons for a nucleic acid library.
89. A method of using a microfluidic device of any one of embodiments 59-86 to sequence a nucleic acid sample.
**90.** A method of using a microfluidic device of any one of embodiments 59-86 to detect a target polynucleotide from a nucleic acid sample.

## Claims

1. A method of generating clusters of amplicons for a nucleic acid library, comprising:
(a) providing a solid surface immobilized with a plurality of forward primers and a plurality of reverse primers, wherein (i) each forward primer comprises, in 5' to 3' order, a 5' region and a 3' region; (ii) each reverse primer comprises, in 5' to 3' order, a 5' region and a 3' region; and (iii) the 5' region of the forward primer and the 5' region of the reverse primer are fully or partially complementary to each other;
(b) contacting the nucleic acid library with the solid surface, wherein each nucleic acid molecule of the library comprises, in 5' to 3' order: (i) a first adapter comprising a sequence that corresponds to the sequence of the 3' region of the reverse primer, a template sequence, and a second adapter comprising a sequence that is complementary to the 3' region of the forward primer; or (ii) a first adapter comprising a sequence that corresponds to the sequence of the 3' region of the forward primer, a template sequence, and a second adapter comprising a sequence that is complementary to the 3' region of the reverse primer; and
(c) performing an amplification process on the solid surface to generate the clusters of amplicons, whereby each nucleic acid molecule from the nucleic acid library is amplified into a clonal cluster of amplicons.

2. The method of claim 1, wherein:
(i) the method further comprises immobilizing the plurality of forward primers and the plurality of reverse primers to the solid surface prior to step (a);
(ii) the forward and/or reverse primers are immobilized to the solid surface at a density of about 1 fmol/mm² to about 1000 fmol/mm², and optionally wherein the forward and/or reverse primers are immobilized to the solid surface at a density of about 200 fmol/mm²;
(iii) the solid surface is a flow cell, a solid bead, a glass slide, or a multi-well plate;
(iv) the 5' region of the forward primer is 3-100 nucleotides in length;
(v) the 5' region of the reverse primer is 3-100 nucleotides in length;
(vi) the 5' region of the forward primer and the 5' region of the reverse primer have the same length;
(vii) the 5' region of the forward primer and the 5' region of the reverse primer have different lengths;
(viii) the 5' region of the forward primer and the 5' region of the reverse primer are completely complementary to each other; or are partially complementary to each other with at least one mismatch; optionally wherein the 5' region of the forward primer and the 5' region of the reverse primer are partially complementary to each other with one, two, or three mismatches;
(ix) the 5' region of the forward primer and/or the 5' region of the reverse primer comprise one or more stretches of identical bases;
(x) each of the one or more stretches comprises at least three identical bases; and/or
(xi) the 5' region of the forward primer is a poly-A strand and the 5' region of the reverse primer is a poly-T strand, or the 5' region of the forward primer is a poly-T strand and the 5' region of the reverse primer is a poly-A strand.

3. The method of claims 1 or 2, wherein:
(i) the 3' region of the forward primer is 20-50 nucleotides in length; optionally, wherein the 3' region of the forward primer is 24 nucleotides in length; further optionally wherein the 3' region of the forward primer has a nucleotide sequence of 5'-CAAGCAGAAGACGGCATACGAGAT-3' (SEQ ID NO: 1); or
(ii) the 3' region of the reverse primer is 20-50 nucleotides in length; optionally wherein the 3' region of the reverse primer is 29 nucleotides in length; further optionally wherein the 3' region of the reverse primer has a nucleotide sequence of 5'-AATGAUACGGCGACCACCGAGATCTACAC-3' (SEQ ID NO: 2).

4. The method of claim 3 , wherein the 5' region of the forward primer is 20 nucleotides in length and the 5' region of the reverse primer is 10 nucleotides in length; optionally
wherein (1) the 5' region of the forward primer consists of 20 A and the 5' region of the reverse primer consists of 10 T, or (2) the 5' region of the forward primer consists of 20 T and the 5' region of the reverse primer consists of 10 A; further optionally
wherein the forward primer has a nucleotide sequence of 5'-AAAAAAAAAAAAAAAAAAAACAAGCAGAAGACGGCATACGAGAT-3' (SEQ ID NO: 3); and the reverse primer has a nucleotide sequence of 5'-TTTTTTTTTTAATGAUACGGCGACCACCGAGATCTACAC-3' (SEQ ID NO: 4).

5. The method of any one of claims 1-4, wherein:
(i) the plurality of forward primers all have the same nucleotide sequence;
(ii) the plurality of reverse primers all have the same nucleotide sequence;
(iii) the plurality of forward primers and the plurality of reverse primers are immobilized to the solid surface adjacent to each other;
(iv) the number of forward primers and the number of reverse primers are at a ratio of from about 1:20 to about 20:1, from about 1:9 to about 9:1, or from about 1:2 to about 2: 1;
(v) the number of forward primers and the number of reverse primers are at a ratio of about 1: 1;
(vi) the method further comprises preparing the nucleic acid library prior to step (b); optionally wherein preparing the nucleic acid library comprises: (i) obtaining nucleic acids from a biological sample, (ii) fragmenting the nucleic acids to proper sizes, and (iii) adding adapters to both ends of the nucleic acid fragments such that the adapter at one end comprises a sequence that corresponds to or is complementary to the 3' region of the forward primer, and the adapter at the other end comprises a sequence that corresponds to is complementary to the 3' region of the reverse primer;
(vii) the nucleic acid library comprises at least two nucleic acid molecules that have neither the same nor the complementary sequence;
(viii) the nucleic acid library is a DNA library.
(ix) the DNA library comprises single-strand DNA molecules; or double-strand DNA molecules;
(x) the DNA library is a cDNA library or a genomic DNA library;
(xi) the amplification process is a polymerase chain reaction (PCR) or a recombinase polymerase amplification (RPA);
(xii) the method further comprises denaturing the nucleic acid library prior to step (c);
(xiii) step (c) comprises:
(1) annealing the nucleic acid molecules from the library to the immobilized forward or reverse primers, (2) generating immobilized DNA strands that are complementary to the nucleic acid molecules from the library by extending the forward or reverse primers, (3) denaturing the double-strand nucleic acids, and (4) washing away the nucleic acid molecules that are not immobilized to the solid surface;
(xiv) step (c) further comprises one or more cycles of bridge PCR during which the immobilized DNA strand extended from the forward primer hybridizes with the reverse primer to generate the complementary strand, and/or the immobilized DNA strand extended from the reverse primer hybridizes with the forward primer to generate the complementary strand;
(xv) the one or more cycles of bridge PCR generate: (1) an immobilized DNA strand extended from the immobilized forward primer which comprises, in 5' to 3' order, the 5' region of the forward primer, a sequence that corresponds to or is complementary to a nucleic acid molecule of the nucleic acid library, and a sequence that is complementary to the 5' region of the reverse primer or to the 3' portion of the 5' region of the reverse primer; and (2) an immobilized DNA strand extended from the immobilized reverse primer which comprises, in 5' to 3' order, the 5' region of the reverse primer, a sequence that corresponds to or is complementary to a nucleic acid molecule of the DNA library, and a sequence that is complementary to the 5' region of the forward primer or to the 3' portion of the 5' region of the forward primer;
(xvi) step (c) further comprises a supersurface PCR during which 3' end of the immobilized DNA strand extended from the forward primer hybridizes with 3' end of the immobilized DNA strand extended from the reverse primer, and each of the two immobilized DNA strands extends at its 3' end using the other immobilized DNA strand as a template;
(xvii) step (c) comprises 5 to 75 cycles of PCR; and/or
(xviii) step (c) comprises 15 to 50 cycles of PCR; optionally wherein the average cluster signal intensity is about 35-50 after 15 cycles of PCR.

6. The method of any one of claims 1-5, further comprising,
(i) after step (c):
(d) incubating the solid surface with an exonuclease for a period of time; and
(e) conducting a second phase of polymerase chain reaction (PCR) to generate the clusters of amplicons; optionally wherein the exonuclease is selected from exonuclease I, exonuclease T, exonuclease P1, and exonuclease VII;
(ii) wherein unused forward primers and reverse primers are removed from the solid surface by the exonuclease at step (d);
(iii) wherein step (e) comprises a supersurface PCR during which 3' end of the immobilized DNA strand extended from the forward primer hybridizes with 3' end of the immobilized DNA strand extended from the reverse primer, and each of the two immobilized DNA strands extends at the 3' end using the other immobilized DNA strand as a template;
(iv) wherein step (e) comprises 5-50 cycles of PCR;
(v) wherein step (e) comprises 25 cycles of PCR'
(vi) wherein step (c) and step (e) comprise a total of 30-60 cycles of PCR;
(vii) wherein step (c) comprises 15 cycles of PCR and step (e) comprises 25 cycles of PCR; optionally wherein the average cluster signal intensity is about 75-100 after 15 cycles of PCR at step (c) and 25 cycles of PCR at step (e); and/or
(viii) wherein the method further comprises sequencing the clusters of amplicons to obtain sequence information for the nucleic acid library; or hybridizing the clusters of amplicons with a probe to detect a target polynucleotide from the nucleic acid library.

7. A method of sequencing a nucleic acid sample comprising:
(a) preparing a nucleic acid library from the nucleic acid sample;
(b) generating clusters of amplicons using methods of any one of claims 1-6;
(c) sequencing the clusters of amplicons to obtain sequence information for the nucleic acid sample.

8. A method of detecting a target polynucleotide from a nucleic acid sample comprising:
(a) preparing a nucleic acid library from the nucleic acid sample;
(b) generating clusters of amplicons using methods of any one of claims 1-6;
(c) hybridizing the clusters of amplicons with a probe to detect a target polynucleotide from the nucleic acid sample.

9. The method of claims 7 or 8, wherein the nucleic acid sample is a total RNA sample or an mRNA sample, and step (a) comprises preparing a cDNA library from the total RNA or the mRNA sample.

10. A microfluidic device comprising a solid surface immobilized with a plurality of forward primers and a plurality of reverse primers, wherein (i) each forward primer comprises, in 5' to 3' order, a 5' region and a 3' region; (ii) each reverse primer comprises, in 5' to 3' order, a 5' region and a 3' region, and (iii) the 5' region of the forward primer and the 5' region of the reverse primer are fully or partially complementary to each other.

11. The microfluidic device of claim 10, wherein:
(i) the solid surface is a flow cell;
(ii) the 5' region of the forward primer is 3-100 nucleotides in length;
(iii) the 5' region of the reverse primer is 3-100 nucleotides in length;
(iv) the 5' region of the forward primer and the 5' region of the reverse primer have the same length;
(v) the 5' region of the forward primer and the 5' region of the reverse primer have different lengths;
(vi) the 5' region of the forward primer and the 5' region of the reverse primer are completely complementary to each other;
(vii) the 5' region of the forward primer and the 5' region of the reverse primer are partially complementary to each other with at least one mismatch; optionally wherein the 5' region of the forward primer and the 5' region of the reverse primer are partially complementary to each other with one, two, or three mismatches;
(viii) the 5' region of the forward primer and/or the 5' region of the reverse primer comprise one or more stretches of identical bases;
(ix) each of the one or more stretches comprises at least three identical bases; and/or
(x) wherein (1) the 5' region of the forward primer is a poly-A strand and the 5' region of the reverse primer is a poly-T strand, or (2) the 5' region of the forward primer is a poly-T strand and the 5' region of the reverse primer is a poly-A strand.

12. The microfluidic device of claims 10 or 11, wherein:
(i) the 3' region of the forward primer is 20-50 nucleotides in length; optionally wherein the 3' region of the forward primer is 24 nucleotides in length; further optionally wherein the 3' region of the forward primer has a nucleotide sequence of 5'-CAAGCAGAAGACGGCATACGAGAT-3' (SEQ ID NO: 1);
(ii) the 3' region of the reverse primer is 20-50 nucleotides in length; optionally wherein the 3' region of the reverse primer is 29 nucleotides in length; further optionallywherein the 3' region of the reverse primer has a nucleotide sequence of 5'-AATGAUACGGCGACCACCGAGATCTACAC-3' (SEQ ID NO: 2);
(iii) the 5' region of the forward primer is 20 nucleotides in length and the 5' region of the reverse primer is 10 nucleotides in length; optionally wherein (1) the 5' region of the forward primer consists of 20 A and the 5' region of the reverse primer consists of 10 T, or (2) the 5' region of the forward primer consists of 20 T and the 5' region of the reverse primer consists of 10 A; further optionally wherein the forward primer has a nucleotide sequence of 5'-AAAAAAAAAAAAAAAAAAAACAAGCAGAAGACGGCATACGAGAT-3' (SEQ ID NO: 3); and the reverse primer has a nucleotide sequence of 5'-TTTTTTTTTTAATGAUACGGCGACCACCGAGATCTACAC-3' (SEQ ID NO: 4);
(iv) the plurality of forward primers all have the same nucleotide sequence;
(v) the plurality of reverse primers all have the same nucleotide sequence;
(vi) the plurality of forward primers and the plurality of reverse primers are immobilized to the solid surface adjacent to each other;
(vii) the number of forward primers and the number of reverse primers are at a ratio of from about 1:20 to about 20:1, from about 1:9 to about 9:1, or from about 1:2 to about 2:1;
(viii) the number of forward primers and the number of reverse primers are at a ratio of about 1:1; and/or
(ix) the forward and/or reverse primers are immobilized to the solid surface at a density of about 1 fmol/mm² to about 1000 fmol/mm²; optionally wherein the forward and/or reverse primers are immobilized to the solid surface at a density of about 200 fmol/mm².

13. A method of producing a microfluidic device of any one of claims 10-12.

14. A method of using a microfluidic device of any one of claims 10-12 to generate clusters of amplicons for a nucleic acid library.

15. A method of using a microfluidic device of any one of claims 10-12 to sequence a nucleic acid sample; or to detect a target polynucleotide from a nucleic acid sample.
